(19) Europäisches Patentamt
European Patent Office
Office européen des brevets

(11) Veröffentlichungsnummer: **0 382 106 B1**

(12) **EUROPÄISCHE PATENTSCHRIFT**

(45) Veröffentlichungstag der Patentschrift: **07.06.95**

(51) Int. Cl.[6]: **C25B 3/04**

(21) Anmeldenummer: **90102043.8**

(22) Anmeldetag: **02.02.90**

(54) **Verfahren zur Herstellung von Thiophenderivaten.**

(30) Priorität: **06.02.89 DE 3903420**

(43) Veröffentlichungstag der Anmeldung:
**16.08.90 Patentblatt 90/33**

(45) Bekanntmachung des Hinweises auf die Patenterteilung:
**07.06.95 Patentblatt 95/23**

(84) Benannte Vertragsstaaten:
**BE CH DE FR GB LI**

(56) Entgegenhaltungen:
**US-A- 4 588 484**

(73) Patentinhaber: **HOECHST AKTIENGESELLSCHAFT**
**D-65926 Frankfurt (DE)**

(72) Erfinder: **Dapperheld, Steffen, Dr.**
**IM Birkenfeld 28**
**D-6238 Hofheim am Taunus (DE)**
Erfinder: **Feldhues, Michael, Dr.**
**Im Weiber 18**
**D-6232 Bad Soden am Taunus (DE)**
Erfinder: **Litterer, Heinz, Dr.**
**Hardtstrasse 77**
**D-6208 Bad Schwalbach (DE)**
Erfinder: **Sistig, Frank Peter, Dr.**
**Junghainzehecken 43**
**D-6238 Hofheim am Taunus (DE)**
Erfinder: **Wegener, Peter, Dr.**
**Am Eichkopf 4**
**D-6240 Königstein/Taunus (DE)**

EP 0 382 106 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf ein elektrochemisches Verfahren zum Austausch von Halogenatomen gegen Wasserstoff- oder Deuteriumatome in Thiophenderivaten.

Halogenierte Thiophenderivate finden breite Verwendung als Zwischenprodukte zur Herstellung von Pharmazeutika und Pflanzenschutzmitteln.

Während die Halogenierung von Thiophen und Thiophenderivaten in der 2- und 5-Position einfach gelingt und auch vollständig halogenierte Thiophenderivate leicht zu erhalten sind, ist die Herstellung von halogenierten Thiophenderivaten mit freier 2- und/oder 5-Position aufwendig.

Im allgemeinen gelingt die Synthese dieser Thiophenderivate durch Isomerisierung halogenierter Thiophenderivate an Katalysatoren oder durch Abspaltung der Halogenatome in der 2- und 5-Position des Thiophenringes höher halogenierter Ausgangsmaterialien unter Verwendung von Reduktionsmitteln oder metallorganischen Verbindungen. Durch Isomerisierung von halogenierten Thiophenderivaten an aciden Katalysatoren erhält man 3-substituierte Thiophene als Gleichgewichtsmischungen mit den Ausgangsverbindungen. Die Isomerisierung wird bei vergleichsweise hohen Temperaturen durchgeführt und die erhaltene Isomerenmischung anschließend aufgetrennt (vgl. Angew. Chem. Int.Ed. 26 (1987), 468). Geeignete Reduktionsmittel sind z.B. unedle Metalle wie Zink, das in Eisessig zur Enthalogenierung von 2,3,5-Tribromthiophen zu 3-Bromthiophen dient (vgl. Organic Synthesis 5 (1973), 149).

Der Einsatz von Metallen zur Enthalogenierung ist mit einem Zwangsanfall von Metallsalzen verbunden, die entsorgt werden müssen. Zusätzlich ist die Selektivität der Enthalogenierung von Thiophenderivaten mit unterschiedlichen (Halogen)-Substituenten unbefriedigend, wodurch die Verwendbarkeit des Verfahrens eingeschränkt wird.

Auch die katalytische Dehalogenierung von chlorierten Thiophenderivaten ist mit der Bildung von Salzen belastet, da äquimolare Mengen an Basen zugesetzt werden müssen. In der GB-PS 1 191 088 ist die Dehalogenierung von chlorierten Thiophenen an Edelmetall-Katalysatoren der Gruppe VIII b des Periodensystems beschrieben, die neben der Verwendung einer Base auch noch hohe Temperaturen und hohe Drücke erfordert.

Ein Verfahren ohne den Zwangsanfall von Salzen, das bei Atmosphärendruck und niedrigen Temperaturen durchgeführt wird, ist die elektrochemische Debromierung von 2,3,5-Tribromthiophen zu 3-Bromthiophen.

In geteilten Elektrolysezellen wird an Kathoden aus Graphit, Blei, Quecksilber oder Zink, in Katholyten aus NaBr in Dioxan/Wasser und bei Stromdichten von maximal 50 $mA/cm^2$ zu Beginn der Elektrolyse 3-Bromthiophen erhalten. Dieses Verfahren ist zur selektiven Dehalogenierung von Tetrabromthiophen zu 3,4-Dibromthiophen ungeeignet, da nur das unerwünschte 3-Bromthiophen entsteht. Auch die selektive Debromierung von 3-Methyl-2,3,4-tribromthiophen entweder zu 3-Methyl-2,4-dibromthiophen oder zu 3-Methyl-4-brom-thiophen gelingt nicht.

3-Methylthiophen entsteht als Produkt der vollständigen Debromierung (vgl. J. Appl. Elektrochem. 10 - (1980), 575).

Die Verwendung eines Glasdiaphragmas zur Trennung von Anoden- und Kathodenraum hat in diesem Verfahren weitere Nachteile, da die in der Technik häufig eingesetzten Durchflußzellen nicht verwendet werden können. Diaphragmen bewirken in Elektrolytsystemen, die mit einer in der Technik üblichen Geschwindigkeit von ca. 0,4 m/s bewegt werden, keine vollständige Trennung von Anolyt und Katholyt, so daß es zur Vermischung der Elektrolyten kommen kann. Die Folge davon wäre, daß im Fall der Oxidation von Bromid-Ionen im Anodenraum gelöstes Brom durch das Diaphragma in den Kathodenraum gelangte, um dort an der Kathode zu Bromid reduziert zu werden. Dies kann zu einer Verminderung der Stromausbeute der Kathodenreaktion führen und damit die Wirtschaftlichkeit des Verfahrens deutlich vermindern.

Die Wirtschaftlichkeit des beschriebenen Verfahrens wird zudem noch durch die in der Technik ungebräuchliche, technisch aufwendige und teure Kontrolle des Elektrodenpotentials herabgesetzt.

Es ist weiterhin die elektrochemische Reduktion von 2-Jod-5-nitrothiophen und 2-Brom-5-nitrothiophen in trockenem Dimethylformamid an Pt-Kathoden und unter Potentialkontrolle beschrieben (vgl. Chemistry of Heterocyclic Compounds (1981), 137). Als Produkte werden 2-Nitrothiophen und bis zu 20 % 2,2′-Dinitro-5,5′-dithienyl gefunden.

Dieses Verfahren ist also unselektiv und durch die Verwendung des teuren Lösemittels DMF, des teuren Elektrodenmaterials Platin und der technisch aufwendigen Potentialkontrolle unwirtschaftlich.

Weiterhin ist die elektrochemische Reduktion von 4,5-Dimethylthiophen-2-carbonsäure zu 2,5-Dihydro-4,5-dimethyl-thiophen-2-carbonsäure bekannt (vgl. Chem. Abstr. 91 (1979), 157538h). Es findet eine partielle Hydrierung des Thiophengrundkörpers statt, die im Falle der Dehalogenierung von halogenierten Thiophenderivaten zu einer nicht unerheblichen Minderung der Ausbeute führen würde.

Aus dem Stand der Technik ergab sich daher die Aufgabe, ein technisch durchführbares und wirtschaftliches Verfahren zur selektiven Dehalogenierung von halogenierten Thiophenderivaten zu finden.

Die Erfindung betrifft somit ein Verfahren zur Herstellung von Thiophenderivaten der Formel I

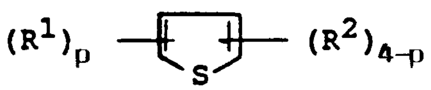 (I),

worin

$R^1$ gleich oder verschieden eine $C_1$-$C_{12}$-Alkyl-, Cycloalkyl-, $C_6$-Aryl-, $C_1$-$C_{12}$-Alkoxy-, Cycloalkoxy-, $C_6$-Aryloxy-, $C_1$-$C_{12}$-Alkylamino-, halogenierte $C_1$-$C_{12}$-Alkyl-, ein Halogenatom oder eine CO-$R^3$-Gruppe, $R^3$ ein Wasserstoffatom, eine OH-, OD-, $C_1$-$C_6$-Alkyl, $C_1$-$C_{12}$-Alkoxy- oder $OR^4$-Gruppe, $R^4$ ein Alkali-, Erdalkali- oder Ammonium-Ion oder eine $(R^5)_4N^+$-Gruppe, $R^5$ ein Wasserstoffatom oder eine $C_1$-$C_6$-Alkyl-Gruppe,

$R^2$ ein Wasserstoff- oder Deuteriumatom,

p null oder eine ganze Zahl von 1 bis 3 bedeutet,

aus einer am kern teilweise oder vollständig chlorierten und/oder bromierten Verbindung der Formel II

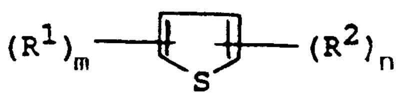 (II),

worin

$R^1$ und $R^2$ die vorgenannte Bedeutung haben und

m eine ganze Zahl von 1 bis 4 und

n null oder eine ganze Zahl von 1 bis 3 bedeuten, und m und n = 4 sind

durch elektrochemische Reaktion, das dadurch gekennzeichnet ist, daß man die elektrochemische Reaktion kontinuierlich oder diskontinuierlich in einer geteilten Elektrolysezelle in einer Elektrolysenflüssigkeit, bestehend aus Wasser oder einem oder mehreren organischen Lösemitteln und aus 1 bis 70 % einer Verbindung der Formel II, bezogen auf die Gesamtmenge des Katholyten, in Gegenwart mindestens einer Onium-Verbindung oder einer Verbindung, die im Elektrolyten in eine Onium-Verbindung umgewandelt wird, die mindestens ein Stickstoff- oder Phosphoratom enthält, und in Gegenwart von mindestens einem löslichen Salz eines Metalles, mit einer Wasserstoffüberspannung größer als 0,25 V, bezogen auf eine Stromdichte von 100 $mA/cm^2$, an einer Kathode mit einer mittleren bis hohen Wasserstoffüberspannung bei einer Stromdichte von 10 bis 500 $mA/cm^2$ und bei einer Temperatur von 0 °C bis zur Siedetemperatur des Elektrolyten durchführt.

In dieser elektrochemischen Reaktion ist $R^1$ vorzugsweise eine $C_1$-$C_6$-Alkylgruppe oder ein Halogenatom, insbesondere eine $C_1$-$C_3$-Alkylgruppe oder ein Halogenatom.

$R^3$ ist vorzugsweise eine OH-, OD-, $C_1$-$C_3$-Alkyl- oder $C_1$-$C_6$-Alkoxy-Gruppe, ein $Na^+$-, $K^+$-, $NH_4{}^+$- oder $(CH_3)_4N^+$-Ion.

Ausgangsverbindungen der Formel II sind mehrfach halogenierte Thiophenderivate, vorzugsweise vollständig oder teilweise bromierte und/oder chlorierte Verbindungen, die sich beispielsweise von den folgenden Substanzen ableiten:

Thiophen, 2- oder 3-Methylthiophen, 2- oder 3-Acetylthiophen, 2,4-Dimethylthiophen, 2- oder 3-Thiophenaldehyd, 2- oder 3-Thiophencarbonsäuren in vollständig oder teilweise chlorierter oder bromierter Form bzw. deren Methyl-, Ethyl- oder Propylester, Natrium-, Kalium- oder Ammonium-Salze.

Die Verbindungen der Formel II werden in einer Konzentration von 1 % bis 70 Gew.-%, vorzugsweise 5 bis 60 Gew.-%, insbesondere 5 bis 50 Gew.-%, bezogen auf die Gesamtmenge des Katholyten einer elektrochemischen Zelle, eingesetzt.

Das erfindungsgemäße Verfahren läßt sich in einer geteilten Elektrolysezelle bei einer Temperatur von 0 °C bis zur Siedetemperatur des Elektrolyten, vorzugsweise von 10 bis 90 °C, insbesondere von 20 bis 80 °C, bei einer Stromdichte von 1 bis 500 $mA/cm^2$, vorzugsweise 20 bis 400 $mA/cm^2$, an einer Kathode aus Blei, Cadmium, Zink, Kupfer, Zinn, Quecksilber oder aus einer Legierung dieser Metalle oder aus Kohlenstoff in einer Elektrolytflüssigkeit, deren flüssiges Medium aus Wasser bzw. einem oder mehreren organischen Lösemitteln besteht und Wasser enthalten kann, durchführen. Zur Teilung der Zellen in den

Anoden- und Kathodenraum lassen sich die üblichen, im Elektrolyten stabilen Diaphragmen aus organischen Polymeren wie Polyethylen, Polypropylen, Polyestern und Polysulfonen, insbesondere halogenhaltigen Polymeren, wie Polyvinylchlorid oder Polyvinylidenfluorid, vorzugsweise aber aus perfluorierten Polymeren, oder aus anorganischen Werkstoffen, wie Glas oder Keramik, vorzugsweise aber Ionenaustauschermembranen, verwenden. Bevorzugte Ionenaustauschermembranen sind Kationenaustauschermembranen aus Polymeren wie Polystyrol, vorzugsweise aber aus perfluorierten Polymeren, die Carboxyl- und/oder Sulfonsäuregruppen enthalten. Die Verwendung von stabilen Anionenaustauschermembranen ist ebenfalls möglich.

Die Elektrolyse kann sowohl kontinuierlich als auch diskontinuierlich und in allen üblichen Elektrolysezellen, wie beispielsweise in Becherglas- oder Platten- und Rahmenzellen oder Zellen mit Festbett- oder Fließbettelektroden, durchgeführt werden. Es ist sowohl die monopolare als auch die bipolare Schaltung der Elektroden anwendbar. Besonders zweckmäßig ist eine Arbeitsweise in geteilten Elektrolysezellen (d.i. mit einer Katholyt- und Anolytflüssigkeit) mit diskontinuierlicher Ausführung der Kathodenreaktion und kontinuerlichem Betrieb der Anodenreaktion.

Erfindungsgemäß werden Kathoden verwendet, die im Elektrolyten stabil sind. Die verwendeten Elektrodenmaterialien haben eine mittlere bis hohe Wasserstoffüberspannung. Bevorzugt ist die Verwendung von Kohlenstoffkathoden, insbesondere bei der Elektrolyse in sauren Elektrolyten mit einem pH-Wert unter 4, da einige der aufgeführten Elektrodenmaterialien, z. B. Zn, Sn, Cd und Pb, Korrosion erleiden können.

Als Kohlenstoffkathoden können alle bekannten Kohlenstoff-Elektrodenmaterialien Verwendung finden, z.B. Elektrodengraphite, imprägnierte Graphitwerkstoffe, poröse Graphite, Kohlefilze, glasartiger Kohlenstoff und auch Kohlenstoff-Kunststoffverbundwerkstoffe. Als Kunststoffe werden bei den Verbundwerkstoffen z.B. Polytetrafluorethylen oder Polyvinylidenfluorid eingesetzt.

Als Anodenmaterial können alle bei Anodenreaktionen üblichen Materialien verwendet werden. Beispiele sind Blei, Bleidioxid auf Blei oder anderen Trägern, Platin oder Edelmetalloxide, z. B. Rutheniumoxid, dotiertes Titandioxid auf Titan oder andere Materialien für die Sauerstoffentwicklung aus verdünnten Säuren wie Schwefelsäure, Phosphorsäure oder Tetrafluoroborsäure oder Kohlenstoff oder mit Edelmetalloxiden dotiertes Titandioxid auf Titan oder anderen Materialien für die Entwicklung von Halogen aus wäßrigen oder alkoholischen Alkalihalogenid- oder Halogenwasserstoff-Lösungen.

Bevorzugte Anolytflüssigkeiten sind wäßrige oder alkoholische Mineralsäuren oder wäßrige Lösungen ihrer Salze, wie verdünnte Schwefelsäure, Phosphorsäure, Tetrafluoroborsäure, konzentrierte Salzsäure, Bromwasserstoff oder Natriumchlorid-, Ammonium-, Kalium- oder Natriumbromidlösungen, insbesondere aber Bromwasserstoff-, Ammonium-, Natrium- oder Kaliumbromid-Lösungen in Wasser und Methanol.

Als organische Lösemittel geeignet sind z.B. kurzkettige aliphatische Alkohole wie Methanol, Ethanol, n- und iso-Propanol oder die verschiedenen Butanole, Amide wie N,N-Dimethylformamid, N-Methyl-2-pyrrolidinon, Nitrile wie Acetonitril, Propionitril, Ketone wie Aceton und chlorierte Kohlenwasserstoffe, wie Dichlormethan und Chloroform. Geeignet sind aber auch chlorierte aromatische Kohlenwasserstoffe wie Chlorbenzol oder Chlortoluol, sowie flüssige halogenierte Thiophenderivate, die während der Elektrolyse entstehen oder dem Katholyten zugesetzt werden können. Voraussetzung für die Verwendbarkeit ist, daß sie unter den Reaktionsbedingungen nicht enthalogeniert werden, leicht abtrennbar und stabil sind. Bevorzugt sind Methanol, Ethanol, die verschiedenen Propanole und Dichlormethan und halogenierte Thiophenderivate, beispielsweise 3-Bromthiophen, 3,4-Dibromthiophen, 3-Brom-4-chlorthiophen, 4-Chlorthiophen, 3,4-Dichlorthiophen, 3-Brom-4-methylthiophen, 3-Brom-2-methylthiophen, 3-Brom-2,4-dimethylthiophen. Auch Gemische können verwendet werden.

Auch eine Zweiphasenelektrolyse unter Zusatz eines nicht wasserlöslichen organischen Lösemittels wie t-Butyl-methylether oder Methylenchlorid in Verbindung mit einem wäßrigen Elektrolyten und einem Emulgator ist möglich.

Weiterhin können dem Katholyten Salze von Metallen mit einer Wasserstoffüberspannung von mindestens 0,25 V (bezogen auf eine Stromdichte von 100 $mA/cm^2$) und/oder enthalogenierenden Eigenschaften zugesetzt werden. Als Salze kommen hauptsächlich die löslichen Salze von Cu, Ag, Au, Zn, Cd, Hg, Sn, Pb, Tl, Ti, Zr, Bi, V, Ta, Cr, Ce, Co oder Ni in Frage, vorzugsweise die löslichen Pb-, Zn-, Sn, Cd- und Tl-Salze. Die bevorzugten Anionen dieser Salze sind $Cl^-$, $Br^-$, $SO_4{}^{2-}$, $NO_3{}^-$ und $CH_3COO^-$.

Bei der Wahl der Anionen ist darauf zu achten, daß mit den Kationen der vorgenannten Metalle keine im Elektrolyten unlöslichen Verbindungen gebildet werden.

Die Salze werden der Elektrolyselösung zugesetzt oder auch, z.B. durch Zugabe von Oxiden, Carbonaten etc. - in einigen Fällen auch der Metalle selbst (sofern löslich) - in der Lösung erzeugt. Ihre Konzentration im Elektrolyten der ungeteilten Zelle sowie im Katholyten der geteilten Zelle wird zweckmäßig auf etwa $10^{-3}$ bis 10 Gew.%, vorzugsweise auf etwa $10^{-2}$ bis 5 Gew.-%, jeweils bezogen auf die Gesamtmenge des Elektrolyten oder Katholyten, eingestellt.

Außerdem werden dem Elektrolyten bzw. dem Katholyten eine oder mehrere Verbindungen mit mindestens einem Stickstoff- oder Phosphoratom gemäß den Formeln III bis VII

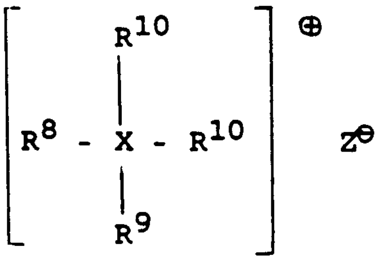

(III)

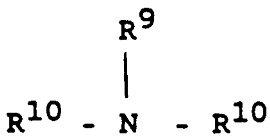

(IV)

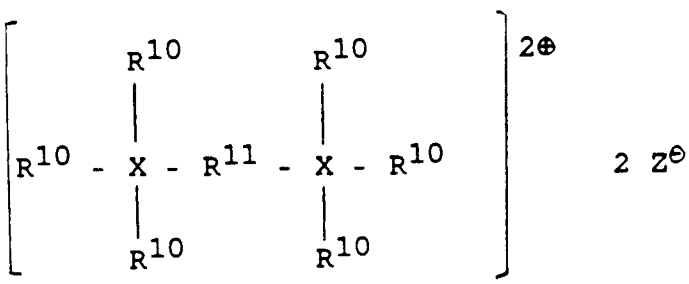

(V)

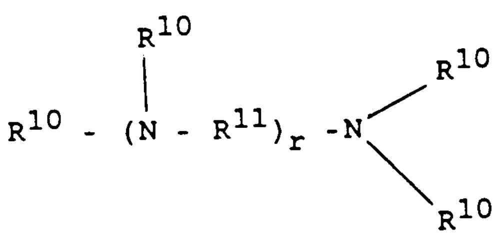

(VI)

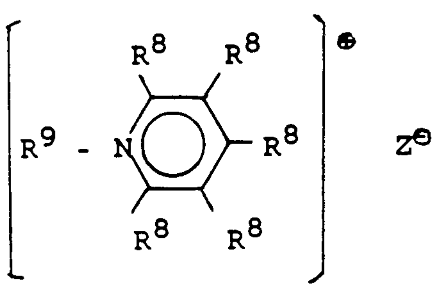

(VII)

zugesetzt,
worin

X Phosphor oder Stickstoff,

$R^8$ Wasserstoff, Alkyl, Cycloalkyl, Aralkyl mit 1 bis 18 C-Atomen im Alkylrest und Aryl mit 6 bis 12 C-Atomen bedeuten.

$R^9$ ist gleich $R^8$ oder eine $(R^8\text{-}O)_q$ $R^8$-Gruppe und q eine ganze Zahl von 1 bis 12.

$R^{10}$ ist gleich $R^8$ oder $R^9$ oder bedeutet eine $-(CH_2Y)_r-CH_2$-Gruppe, wobei r null oder eine ganze

Zahl von 1 bis 6, Y -NH, Sauerstoff, Schwefel oder $-CH_2$-ist.

$R^{11}$ ist $-(CH_2)_r-$,- $CH_2-[O-(CH_2)_r]_r-O-(CH_2)_2-$ und

Z eine OH-Gruppe oder ein Anion einer anorganischen oder organischen Säure. Diese Säuren sind beispielsweise die verschiedenen Halogenwasserstoffsäuren, Schwefelsäure, Salpetersäure, salpetrige Säure, Phosphorsäure, $H_3BO_3$, $HBF_4$, $HPF_6$, Ameisensäuren, Essigsäure, Oxalsäure.

Bei den Verbindungen I bis VII handelt es sich um Onium-Verbindungen bzw. um Verbindungen die im Elektrolyten in eine Onium-Verbindung umgewandelt werden.

Die Verbindungen der Formel II bis VII werden in Konzentrationen von $10^{-3}$ bis 10 Gew.-%, vorzugsweise $10^{-2}$ bis 5 Gew.-%, bezogen auf den Elektrolyten bzw. Katholyten, zugesetzt.

Es eignen sich insbesondere Salze mit den Kationen Tetramethyl-, Tetraethyl-, Tetrapropyl-, Tetrabutylammonium oder -phosphonium, Benzyl-, Octyl-, Decyl-, Dodecyl-, Tetradecyl-, Hexadecyl-, Octadecyltrimethylammonium oder -trimethylphosphonium, Dioctyl-, Didecyl-, Didodecyl-, Ditetradecyl-, Dihexadecyl-, Dioctadecyldimethylammonium oder -dimethylphosphonium, Methyltrioctylammonium, N-Hexadecylpyridinium und Kombinationen. Es können aber auch primäre, sekundäre und tertiäre Amine eingesetzt werden, aus denen im Verlauf der Elektrolyse die Oniumverbindungen gebildet werden. Die Art der Anionen der Onium-Verbindungen ist für das Verfahren ohne Belang, bevorzugt werden die Halogenid-, Sulfat-, Tetrafluorborat- und Hydroxid-Ionen.

Zur Erhöhung der Leitfähigkeit können dem Katholyten anorganische oder organische Säuren zugesetzt werden, vorzugsweise Säuren wie Chlor- oder Bromwasserstoffsäure und Schwefelsäure sowie Essigsäure. Bei der Verwendung von Säuren, die mit den obengenannten Metallen im neutralen oder basischen Bereich schwer lösliche Verbindungen bilden, wird natürlich nur in solchen pH-Bereichen gearbeitet, in denen sich keine unlöslichen Verbindungen bilden.

Auch die Zugabe von Basen kann zur Einstellung des für die Elektrolyse günstigen pH-Wertes nötig sein und den Verlauf der Elektrolyse günstig beeinflussen. Geeignet sind primäre, sekundäre und tertiäre $C_2$-$C_{12}$-Alkyl- und Cycloalkylamine, aromatische und aliphatisch-aromatische (insbesondere araliphatische) Amine und deren Salze, anorganische Basen wie Alkali- und Erdalkalihydroxide, beispielsweise Li-, Na-, K-, Cs-, Mg-, Ca- oder Ba-hydroxid und Gemische.

Die Aufarbeitung des Katholyten kann durch Destillation auf übliche Weise erfolgen. Besonders vorteilhaft ist es, den Elektrolyten bzw. Katholyten mit Wasser zu versetzen, um die Löslichkeit der Produkte zu vermindern. Sie können so nach der Phasentrennung einfach isoliert werden. Die verbleibende wäßrige Lösung wird zur Abtrennung von Produktresten extrahiert und destillativ in ihre Bestandteile aufgetrennt. Die organischen Lösemittel, wäßrige Säuren, die zugesetzen Metallsalze und die Verbindungen der Formeln II-VI können in die Elektrolyse zurückgeführt werden. Bei brom- oder chlorwasserstoffhaltigen Lösungen kann auf die Destillation verzichtet werden, sie sind als Teil des Anolyten direkt zu verwenden.

Das erfindungsgemäße Verfahren wird durch die folgenden Beispiele erläutert.

Die nach dem Stand der Technik in einer durch eine Kationenaustauschermembran geteilten Umlaufzelle und unter Verzicht auf die Kontrolle des Elektrodenpotentials durchgeführte Elektrolyse ergab im Vergleichsbeispiel A nicht das gewünschte Produkt bzw. im Vergleichsbeispiel B verlief die Reaktion nicht selektiv und lieferte einen hohen Anteil an vollständig dehalogeniertem Produkt.

In beiden Beispielen war die Löslichkeit der Ausgangsverbindungen im Katholyten, der aus einer 0,2 molaren Lösung Natriumbromid in Dioxan/Wasser (70:30) bestand, so schlecht, daß in gesättigten Lösungen gearbeitet werden mußte, was in einem Fall zur Verstopfung der Elektrolysezelle führte.

In beiden Fällen stieg der pH-Wert des Katholyten im Verlauf der Elektrolyse auf Werte größer als 12; es bestand also die Gefahr, daß unerwünschte Reaktionen am Thiophenkörper oder einem Substituenten ablaufen könnten.

Schließlich erlitt die an sich korrosionsstabile Graphit-Kathode unter den Bedingungen der Vergleichsbeispiele Korrosionsschäden, so daß eine Verwendung dieser Methode in der Technik nicht sinnvoll erscheint.

Für die nachfolgenden Beispiele wurden zwei Arten von Elektrolysezellen verwendet.

**Elektrolysezelle 1:**

Platten- und Rahmenzelle mit 0,02 $m^2$ Elektrodenfläche. Als Kathode wurde Elektrodengraphit oder imprägnierter Graphit (®Diabon N der Fa. Sigri, Meitingen, Deutschland) und als Anode imprägnierter Graphit oder eine Platinplatte verwendet.

Anolyt: wäßrige 15 bis 35 %ige Salzsäure, gesättigte methanolische Salzsäure, methanolische Bromwasserstoff-Lösung oder 0,5 bis 2 n wäßrige Schwefelsäure. Der Elektrodenabstand betrug 4 mm und als Abstandhalter wurden Netze aus Polyethylen verwendet. Die Kationenaustauschermembran war eine Zwei-

oder Einschichtenmembran aus einem Copolymerisat aus einem Perfluorsulfonylethoxyvinylether und Tetrafluorethylen (Typ ®Nafion 324 oder 423 der Fa. Du Pont, Wilmington, Dela., USA). Der Durchfluß des Elektrolyten betrug 400 l/h.

**Elektrolysezelle 2:**

Ummantelte Glastopfzelle mit einem Volumen von 350 ml und einem Kunststoffeinsatz für den Anodenraum.
Kathode: imprägnierter Graphit (®Diabon N der Fa. Sigri, Meitingen, Deutschland);
Anode: Platinnetz, Graphit- oder Bleiplatte (20 $cm^2$);
Kathodenfläche: 12 $cm^2$; Elektrodenabstand: 1,5 cm;
Anolyt: wie in Elektrolysezelle 1;
Kationenaustauschermembran: ®Nafion 324
Stofftransport: durch Magnetrührer.

**Beispiel 1**

Ein Katholyt aus 2 l Methanol, 500 ml Methylenchlorid, 1 g Zinkchlorid, 5 g Tetraethylammoniumbromid und 159 g eines Gemisches aus 3-Methyl-2,4,5-tribromthiophen (95 %) und 3-Brommethyl-2,5-dibromthiophen (5 %) wurde in der Elektrolysezelle 1 bei einer Stromdichte von 100 mA/$cm^2$, einer Spannung von 7 bis 8 V und einer Temperatur von 28 bis 38 °C elektrolysiert. Der Stromverbrauch betrug 53 Ah. Während der Elektrolyse wurde durch portionsweise Zugabe von insgesamt 150 g Natriumhydroxid der pH-Wert des Katholyten auf einem Wert zwischen 5 und 7 gehalten. Nach Zugabe von 2 l Wasser zum Katholyten, Phasentrennung, Extraktion mit $CF_2Cl$-$CFCl_2$ und Abdestillation des Lösemittels wurden 73,67 g 3-Brom-4-methylthiophen (Ausbeute: 89,9 %), 1,47 g 2,4-Dibrom-3-methylthiophen und 2,3-Dibrom-4-methylthiophen (Ausbeute: 1,2 %) und 4,16 g 3-Methylthiophen (Ausbeute: 8,9 %) erhalten.

**Beispiel 2**

Ein Katholyt aus 400 ml Methanol, 200 ml Methylenchlorid, 1 g Zinn(II)-chlorid, 5 g Tetramethylammoniumchlorid und 20,4 g 2-Methyl-3,4,5-tribromthiophen wurde in der Elektrolysezelle 1 bei einer Stromdichte von 25 mA/$cm^2$, einer Spannung vom 3,8 bis 2,9 V und einer Temperatur von 30 °C elektrolysiert. Der Stromverbrauch betrug 8,4 Ah. Nach Zugabe von 500 ml Wasser zum Katholyten, Phasentrennung und Abdestillation des Methylenchlorids erhielt man 12,98 g 3,4-Dibrom-2-methylthiophen (Ausbeute: 83,1 %), 0,45 g eines Gemisches aus 2,3-Dibrom-5-methylthiophen und 2,4-Dibrom-5-methylthiophen (Ausbeute: 2,7 %) und 0,071 g nicht umgesetztes 2-Methyl-3,4,5-tribromthiophen.

**Beispiel 3**

Ein Katholyt aus 1,6 l Methanol, 2 l Methylenchlorid, 100 ml Bromwasserstoff-Lösung (48 % in Wasser), 6 g Tetramethylammoniumchlorid, 0,6 g Bleiacetat • Dihydrat und 400 g 2-Methyl-3,4,5-tribromthiophen in der Elektrolysezelle 1 elektrolysiert.
Die Stromdichte betrug 200 mA/$cm^2$ zu Beginn und 100 mA/$cm^2$ am Ende des Ansatzes. Bei Temperaturen von 35 bis 40 °C stellte sich eine Spannung von 8 bis 7 V ein. Im Verlaufe der Elektrolyse wurden noch 498 g 2-Methyl-3,4,5-tribromthiophen zugegeben. In regelmäßigen Abständen wurden 2 l des Katholyten entnommen, mit Wasser versetzt und die organische Phase in den Katholyten zurückgeführt. Um den dadurch auftretenden Verlust an Leitsalz und Bleisalz auszugleichen, wurden zum Katholyten 6 g Tetramethylammoniumchlorid und 1 g Bleiacetat • Dihydrat zugegeben. Zur Einstellung eines pH-Wertes von 5 bis 7 wurde der Katholyt portionsweise mit insgesamt 450 ml einer 25 prozentigen Lösung von Ammoniak in Wasser versetzt. Der Anolyt bestand aus einer Lösung von 2,5 l Methanol, 500 ml Wasser und 500 ml HBr-Lösung. Es wurde kontinuierlich mit insgesamt 4 l MeOH, 1 l Wasser und 250 ml HBr-Lösung aufgefüllt und Brom entwickelt.
Nach einem Stromverbrauch von 888 Ah wurde gomäß Beispiel 1 aufgearbeitet und man erhielt 5,05 g unumgesetztes 2-Methyl-3,4,5-tribromthiophen, 469 g 3,4-Dibrom-2-methylthiophen (Ausbeute: 68,6 %), 21,3 g eines Gemisches isomerer Dibrommethylthiophene (Ausbeute: 3,1 %) und 27,6 g isomere Brom-2-methylthiophene (Ausbeute: 5,8 %).

**Beispiel 4**

Ein Katholyt aus 200 ml Methanol, 50 ml Methylenchlorid, 5 g Kaliumfluorid und 8,9 g 2-Methyl-3,4,5-tribromthiophen wurde in der Elektrolysezelle 2 an einer Zink-Kathode bei einer Stromdichte von anfangs 83 $mA/cm^2$ und einer Spannung von 20 bis 17 V, nach einem Stromverbrauch von 2,5 Ah bei einer Stromdichte von 42 $mA/cm^2$ und einer Spannung von 13,5 V und bei einer Temperatur von 30 °C bzw. 24 °C elektrolysiert. Der Stromverbrauch betrug insgesamt 6,75 Ah.
Es wurde gemäß Beispiel 1 aufgearbeitet. Man erhielt 0,58 g nicht umgesetztes 2-Methyl-3,4,5-tribromthiophen, 4 g 3,4-Dibrom-2-methylthiophen (Ausbeute: 62,9 %), 0,37 g eines Gemisches isomerer Dibrom-2-methylthiophene (Ausbeute: 5,6 %) und 0,13 g eines Gemisches isomerer Brom-2-methylthiophene (Ausbeute: 0,3 %). Die Kathode wies deutliche Korrosionsspuren auf.

**Beispiel 5**

Ein Katholyt aus 2 l Methanol, 1 l Methylenchlorid, 50 ml 48 %ige Bromwasserstoff-Lösung in Wasser, 20 g Tetraethylammoniumbromid und 341 g 3-Methyl-2,4,5-tribromthiophen (mit ca. 91 % Reinheit, wurde in der Elektrolysezelle 1 bei einer Stromdichte von 160 bis 180 $mA/cm^2$, einer Spannung von 8 V und einer Temperatur von 30 bis 45 °C elektrolysiert.
Der Stromverbrauch betrug 144 Ah. Nach Zugabe von 1 l Wasser zum Katholyten und der gemäß Beispiel 1 beschriebenen Aufarbeitung erhielt man
204 g 2,4-Dibrom-3-methylthiophen (Ausbeute: 80 %),
4,9 g nicht umgesetztes 3-Methyl-2,4,5-tribromthiophen,
4,8 g 2,3-Dibrom-4-methylthiophen (Ausbeute: 1,9 %),
15,4 g 4-Brom-3-methylthiophen (Ausbeute: 8,7 %) und
1,82 g 2-Brom-3-methylthiophen (Ausbeute: 1%).

**Beispiel 6**

Ein Katholyt aus 3 l Methanol, 1 l Methylenchlorid, 2 g Hexadecylpyridiniumchlorid und 455 g 3-Methyl-2,4,5-tribromthiophen, das als Verunreinigung 18 g 2,5-Dibrom-3-methylthiophen und 22 g Wasser enthielt, wurde in der Elektrolysezelle 1 bei einer Stromdichte von 50 $mA/cm^2$ zu Beginn der Elektrolyse, 8 V Klemmenspannung und einer Temperatur von 30 °C elektrolysiert. Innerhalb von 30 Minuten konnte die Stromdichte auf 150 $mA/cm^2$, bei einer Spannung von anfangs 8 V, erhöht werden. Im Verlauf der Elektrolyse sank die Spannung auf 6,1 V.
Der Stromverbrauch betrug 236,4 Ah. Nach zugabe von 3 l Wasser zum Katholyten und der gemäß Beispiel 1 beschriebenen Aufarbeitung erhielt man:
187,4 g 3-Brom-4-methylthiophen (Ausbeute: 85,4 %),
26,4 g 2,4-Dibrom-3-methylthiophen (Ausbeute: 8,4 %) und
10,7 g 2-Brom-3-methylthiophen (Ausbeute: 4,8 %).

**Beispiel 7**

Ein Katholyt aus 400 ml Methanol, 50 ml Methylenchlorid, 0,4 g Bleiacetat • Dihydrat 12 g Tetramethylammoniumchlorid und 53 g 3-Methyl-2,4,5-tribromthiophen wurde in der Elektrolysezelle 1 bei einer Stromdichte von 150 $mA/cm^2$ zu Beginn und 50 $mA/cm^2$ am Ende des Ansatzes, einer Spannung von 9 bis 7 bzw. 4 V und einer Temperatur von 32 bis 35 °C elektrolysiert. Der Stromverbrauch betrug 22 Ah. Nach Zugabe von 800 ml Wasser zum Katholyten, Phasentrennung, Extraktion der wäßrigen Phase mit Pentan, Vereinigung der organischen Phasen und Abdestillation des Lösemittels erhielt man 3,57 g nicht umgesetztes 3-Methyl-2,4,5-tribromthiophen, 19,6 g 3-Brom-4-methylthiophen (Ausbeute: 75,5 %) und 0,88 g 3-Methylthiophen (Ausbeute: 6,1 %).

**Beispiel 8**

Ein Katholyt aus 300 ml Methanol, 100 ml Methylenchlorid, 15 g Zinkchlorid, 0,5 g Methyltrioctylammoniumchlorid und 31 g 3-Methyl-2,4,5-tribromthiophen wurde in der Elektrolysezelle 1 bei einer Stromdichte von 50 $mA/cm^2$ zu Beginn und 25 $mA/cm^2$ am Ende des Ansatzes, einer Spannung von 4 bzw. 3 V, einer Temperatur von 34 bis 24 °C und einem pH-Wert von -0,8 elektrolysiert. Der Stromverbrauch betrug 14,8 Ah. Nach Zugabe von 500 ml Wasser zum Katholyten und der gemäß Beispiel 1 beschriebenen Aufarbeitung erhielt man 17,4 g 2,4-Dibrom-3-methylthiophen (Ausbeute: 73,4 %), 0,71 g 2,3-Dibrom-4-methylthiophen (Ausbeute: 3 %) und 2,92 g 4-Brom-3-methylthiophen (Ausbeute: 17,8 %).

| Beispiel | 9 | 10 | 11 | 12 | 13 | 14 |
|---|---|---|---|---|---|---|
| Elektrolysezelle | 1 | 1 | 1 | 1 | 2 | 2 |
| Kathode | Imprägnierter Graphit | Imprägnierter Graphit | Imprägnierter Graphit | Imprägnierter Graphit | Graphit | Blei 3) |
| Anfangskatholyt | 4 l $CH_3OH$<br>0,5 l $CH_2Cl_2$<br>10 g $(CH_3)_4NCl$<br>1 g $Pb(OCOCH_3)_2 \cdot 2H_2O$<br>100 ml HBr(48% in $H_2O$) | 2,5 l $C_2H_5OH$<br>0,5 l $CH_2Cl_2$ | 1,4 l $CH_2Cl_2$<br>0,6 l $H_2O$<br>60 ml $HBr \cdot H_2O$<br>1 g $(Pb\ (OCOCH_3)_2 \cdot 2H_2O$<br>1 g $CH_3N(C_8H_{15})_3Cl$ | 2 l $CH_3OH$<br>0,5 l $CH_2Cl_2$<br>100 ml $HBr \cdot H_2O$<br>1 g $Pb(OCOCH_3)_2 \cdot 2H_2O$<br>2 g $C_{12}H_{25}NH_2$ | 100 ml $CH_3OH$<br>50 ml $CH_2Cl_2$<br>0,5 g $CdCl_2$ | 100 ml $CH_3OH$<br>50 ml $CH_3CN$<br>10 ml $HBr \cdot H_2O$ |
| Tetrabromthiophen [g] | 570 | 80 | 50 | 50 | 10 | 10 |
| Anode | Graphit | Graphit | Graphit | Graphit | Pt | Pt |
| Anolyt | $CH_3OH \cdot HCl$ | $CH_3OH \cdot HCl$ | $HBr \cdot H_2O$ | $HBr \cdot CH_3OH/H_2O$ 2) | $HBr \cdot CH_3OH$ | $HBr \cdot CH_3OH$ |
| Stromdichte [$mA/cm^2$] | 250 - 100 | 100 | 50 - 200 1) | 100 - 50 | 83 | 83 |
| Spannung [V] | 8 - 4,2 | 7 - 5,5 | 10 - 22 | 6 - 5,5 | 13,5 - 11,5 | 8 - 10 |
| Temperatur [°C] | 33 - 50 | 38 - 42 | 29 - 34 | 32 | 35 | 30 |
| Stromverbrauch [Ah] | 142,4 | 46,4 | 76 | 51,2 | 5,06 | 13,6 |
| Elektrolyse-Ergebnis | | | | | | 4) |
| 2,3,4-Tribromthiophen [g] (Ausbeute) | 23,4 (4,9 %) | 1,4 (2,2 %) | - | - | 0,036 (0,4) | 0,39 (9,2) |
| 3,4-Dibromthiophen | 301 (87,0 %) | 29,5 (61 %) | 10,4 (34,3 %) | 21,85 (71,3 %) | 5,39 (89,2%) | 3,21 (53,2%) |
| 3-Bromthiophen | 9,2 (7,6 %) | 1,5 (4,7 %) | 8,22 (40,3 %) | 2,8 (13,8 %) | 0,056 (1,2%) | 1,14 (14%) |

1) nach Einstellung von pH=9 durch NaOH-Zugabe

2) aufgearbeiteter Katholyt aus Beispiel 11

3) Korrosion der Kathode

4) 7 Nebenprodukte

Die Aufarbeitung erfolgte gemäß Beispiel 1.

**Beispiel 15**

Ein Katholyt aus 2 l Methanol, 50 ml Bromwasserstoff-Lösung (48 % in Wasser), 50 ml Ammoniak-Lösung (25 % in Wasser), 0,6 g Bleiacetat • Dihydrat, 5 g Methyltrioctylammoniumchlorid und 128 g eines Gemisches von bromierten Thiophenen, die bei der Reinigung von Tetrabromthiophen anfallen und zu 3-Bromthiophen bzw. Thiophen umgesetzt werden sollten, bestehend aus 23,24 % Tetrabromthiophen, 35,0 % isomerer Tribromthiophene und 39,5 % isomerer Dibromthiophene, wurden in der Elektrolysezelle 1 elektrolysiert. Der Anolyt bestand aus einer Lösung von Ammoniak in Methanol. Bei einer Stromdichte von 50 mA/cm$^2$ und Temperaturen von 38 bis 56 °C wurde bei einer Spannung von 23 V zu Beginn und 7 V am Ende des Ansatzes eine Ladungsmenge von 122,4 Ah verbraucht. Zur Aufarbeitung wurde der Katholyt mit 2 l Wasser versetzt, mit $CF_2Cl$-$CFCl_2$ extrahiert und der Extrakt mit verdünnter Salzsäure von anhaftendem Ammoniak befreit. Nach Abdestillation des Lösemittels erhielt man 55,75 g 3-Bromthiophen (Ausbeute: 81,8 %) und 4,62 g Thiophen (Ausbeute: 13,1 %).

**Beispiel 16**

Ein Katholyt aus 450 ml Methanol, 150 ml Methylenchlorid, 0,6 g Bleiacetat • Dihydrat, 5 g Tetrabutylphosphoniumbromid und 20 g 2-Chlor-3,4,5-tribromthiophen wurde in der Elektrolysezelle 1 bei einer Stromdichte von 50 mA/cm$^2$ zu Beginn und 25 mA/cm$^2$ am Ende des Ansatzes, bei einer Spannung von 5,1 bis 3 V und einer Temperatur von 29 - 49 °C elektrolysiert. Nach der Zugabe von 500 ml Wasser, Phasentrennung, Extraktion der wäßrigen Phase mit $CF_2Cl$-$CFCl_2$, Vereinigung der organischen Phasen und Abdestillation des Lösemittels erhielt man 8,74 g 2-Chlor-3,4-dibromthiophen (Ausbeute: 56,6 %), 0,6 g eines 2-Chlor-dibromthiophens unbekannter Struktur (Ausbeute: 3,75 %), 2,7 g isomere Dibromthiophene (Ausbeute: 10,8 %) und 0,6 g 2,3,4-Tribromthiophen (Ausbeute: 3,1 %).

**Beispiel 17**

Ein Katholyt aus 650 ml Methanol, 0,5 g Bleiacetat Dihydrat, 0,5 g Tetrabutylammonium-Tetrafluoroborat, 20 ml Bromwasserstoff-Lösung (48 % in Wasser) und 20 g 3-Chlor-2,4,5-tribromthiophen wurde in der Elektrolysezelle 1 bei einer Stromdichte von 100 mA/cm$^2$ zu Beginn und 25 mA/cm$^2$ am Ende des Ansatzes, einer Spannung von 7,2 bis 3,4 V und einer Temperatur von 28 bis 35 °C elektrolysiert. Der Stromverbrauch betrug 8,8 Ah. Nach Zugabe von 100 ml Wasser zum Katholyten, Extraktion mit $CF_2Cl$-$CFCl_2$ und Abdestillieren des Lösemittels erhielt man 8,24 g 4-Brom-3-chlorthiophen (Ausbeute: 73,8 %), 0,665 g 3-Chlorthiophen (Ausbeute: 9,1 %) und 0,4 g Dibromthiophen unbekannter Struktur (Ausbeute: 2,9 %).

**Beispiel 18**

Ein Katholyt aus 4 l Methanol, 1 l Methylenchlorid, 2 g Bleiacetat • Dihydrat, 20 g Tetramethylammoniumchlorid, 100 ml Bromwasserstoff-Lösung (48 % in Wasser) und 300 g eines Gemisches aus 258 g 3-Chlor-2,4,5-tribromthiophen und 42 g Tetrabromthiophen wurde in der Elektrolysezelle 1 bei einer Stromdichte von 200 mA/cm$^2$, einer Spannung von 14 V und einer Temperatur von 38 °C elektrolysiert.

Nach Auflösung des suspendierten Ausgangsprodukts und Bildung von teilweise enthalogenierten, flüssigen Thiophenderivaten, konnte 1600 g Ausgangsprodukt der oben aufgeführten Zusammensetzung portionsweise zugegeben werden.
Die Stromdichte wurde auf 50 mA/cm$^2$ gesenkt und es stellte sich eine Temperatur von 30 - 32 °C bei einer Spannung von 4 bis 4,5 V ein.

Nach einem Ladungsverbrauch von 1318 Ah wurde der Katholyt mit der gleichen Menge Wasser versetzt und das Produkt isoliert.
Man erhielt
872 g 3-Brom-4-chlorthiophen
(Ausbeute: 96,3 %, bezogen auf eingesetztes 3-Chlor-2,4,5-tribromthiophen),
8,86 g 4-Chlor-2,3-dibromthiophen
(Ausbeute: 0,6 %, bezogen auf eingesetztes 3-Chlor-2,4,5-tribromthiophen),
2,69 g 2,3,4-Tribromthiophen,
(Ausbeute: 1,3 %, bezogen auf eingesetztes 3-Chlor-2,4,5-tribromthiophen) und
126,6 g 3,4-Dibromthiophen
(Ausbeute: 78,8 %, bezogen auf eingesetztes Tetrabromthiophen).

**Beispiel 19**

Ein Katholyt aus 200 ml Methanol 5 g Tetrabutylphosphoniumbromid, 0,4 g TlCl und 10 g 2-Acetyl-5-bromthiophen wurde in der Elektrolysezelle 2 bei einer Stromdichte von 83 mA/cm$^2$, einer Spannung von 23 bis 10 V und einer Temperatur von 30 °C elektrolysiert. Der Stromverbrauch betrug 6,3 Ah.
Der Anolyt bestand aus einer Lösung von Schwefelsäure in Methanol, als Anode diente ein Platin-Draht.
Zur Aufarbeitung wurde der Katholyt mit 200 ml Wasser versetzt und mit Pentan extrahiert. Nach Abdestillation des Lösemittels erhielt man 4,28 g 2-Acetylthiophen (Ausbeute: 77,7 %) und 1,04 g unumgesetztes 2-Acetyl-5-bromthiophen.

**Beispiel 20**

Ein Katholyt aus 2 l Methanol, 20 g Tetramethylammoniumchlorid, 0,3 g Bleiacetat•Dihydrat, 50 ml 25 %ige Ammoniak-Lösung in Wasser und 1 kg einer Lösung bestehend aus 0,883 kg 3-Bromthiophene, 0,101 kg 2-Bromthiophen und 0,016 kg Thiophen wurde in der Elektrolysezelle 1 elektrolysiert.

Der Anolyt bestand aus einer Lösung von 10 % Ammoniumbromid in Methanol.
Bis zu einem Ladungsverbrauch von 26 Ah betrug die Stromdichte 200 mA/cm$^2$, bis 115 Ah betrug sie 100 mA/cm$^2$ und mit 50 mA/cm$^2$ wurde bis zu einem Ladungsverbrauch von 131 Ah elektrolysiert.

Bei Temperaturen von 52 bzw. 42 bis 44 °C lag die Spannung bei 16 bzw. 9 bis 11 und schließlich bei 6 V.
Der pH-Wert lag bei 7,7.

Nach Zugabe von 3 l Wasser zum Katholyten und Phasentrennung, sowie Extraktion der wäßrigen Phase mit $CF_2Cl\text{-}CFCl_2$ undAbdestillieren des Lösemittels erhielt man:
0,865 kg 3-Bromthiophen,
0,002 kg 2-Bromthiophen und
0,066 kg Thiophen.

**Vergleichsbeispiel A**

Ein Katholyt aus 1,4 l Dioxan, 600 ml Wasser, 40,8 g Natriumbromid und 50 g Tetrabromthiophen wurde in der Elektrolysezelle 1 bei einer Stromdichte von 150 mA/cm$^2$, einer Spannung von 23 bis 11 V und einer Temperatur von 55 °C elektrolysiert.
Zu Beginn der Elektrolyse war nur ein geringer Teil der Ausgangsverbindung im Elektrolyten löslich, so daß es zur Verstopfung der Elektrolytkanäle in der Elektrolysezelle kam. Nach Erhöhung der Temperatur auf 70 °C und Zugabe der Ausgangsverbindung in kleinen Portionen traten diese Probleme nicht mehr auf. Im Anolyten wurden 2,1 l Dioxan, 900 ml Wasser und 62 g Natriumbromid an einer Graphit-Anode elektrolysiert. Im Verlauf der Elektrolyse wurden noch 100 g Natriumbromid zugegeben. Der Stromverbrauch betrug insgesamt 91,2 Ah, 63 % der Ladungsmenge wurden für die Bildung von Wasserstoff verbraucht.
Im Verlauf der Elektrolyse wurde die Zusammensetzung des Katholyten zweimal analysiert.

| | 1 | 2 |
|---|---|---|
| Stromverbrauch: | 63,2 Ah | 91,2 Ah |
| Tetrabromthiophen | 25,4 % | 6,2 % |
| 2,3,4-Tribromthiophen | 1,7 % | - |
| 3,4-Dibromthiophen | 12,9 % | 2,8 % |
| 3-Bromthiophen | 48,7 % | 90,9 % |

Zur Aufarbeitung wurde gemäß Beispiel 1 vorgegangen. Die Isolierung des Produktes wurde durch schlechte Phasentrennung und stabile Emulsionen erschwert. Nach siebenmaliger Extraktion der Lösung mit $CF_2Cl\text{-}CFCl_2$ und Abtrennung des Lösemittels erhielt man 20 g eines Gemisches aus 4,6 g Dioxan, 14 g 3-Bromthiophen (Ausbeute: 70 %), 0,95 g nicht umgesetztes Tetrabromthiophen und 0,45 g 3,4-Dibromthiophen (Ausbeute: 1,5 %).

**Vergleichsbeispiel B**

Ein Katholyt aus 500 ml Dioxan, 200 ml Wasser, 14,4 g Natriumbromid und 15 g 3-Methyl-2,4,5-tribromthiophen wurde in der Elektrolysezelle 1 bei einer Stromdichte von 50 mA/cm$^2$, einer Spannung von

10,6 bis 9,2 V und einer Temperatur von 28 bis 36 °C an einer Kathode aus Graphit elektrolysiert. An einer Anode aus Platin wurden im Anolyten 20,6 g Natriumbromid in 700 ml Dioxan und 300 ml Wasser oxidiert. Der Stromverbrauch betrug 11,2 Ah und der pH-Wert des Katholyten stieg von 2,4 zu Beginn der Elektrolyse auf 12,5. Im Verlauf der Elektrolyse färbte sich der Katholyt dunkel, was auf suspendierte Graphitteilchen zurückgeführt wurde, die von der Korrosion der Kathode herrührten.

Zur Aufarbeitung wurde wie in Vergleichsbeispiel 1 verfahren. Man erhielt 6,8 g eines Gemisches verschiedener Produkte:

0,66 g nicht umgesetztes 3-Methyl-2,4,5-tribromthiophen,
0,43 g 3-Methyl-2,4-dibromthiophen (Ausbeute: 9,7 %),
4,88 g 4-Brom-3-methylthiophen (Ausbeute: 64,1 %),
0,1 g 2-Brom-3-methylthiophen (Ausbeute: 1,2 %) und
0,7 g des unerwünschten, vollständig enthalogenierten 3-Methylthiophens (Ausbeute: 16,3 %).

**Patentansprüche**

1. Verfahren zur Herstellung von Thiophenderivaten der Formel I

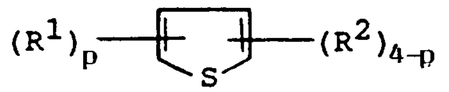

(I),

worin

R¹ gleich oder verschieden eine $C_1$-$C_{12}$-Alkyl-, Cycloalkyl-, $C_6$-Aryl-, $C_1$-$C_{12}$-Alkoxy-, Cycloalkoxy-, $C_6$-Aryloxy-, $C_1$-$C_{12}$-Alkylamino-, halogenierte $C_1$-$C_{12}$-Alkyl-, ein Halogenatom oder eine CO-$R^3$-Gruppe, $R^3$ ein Wasserstoffatom, eine OH-, OD-, $C_1$-$C_6$-Alkyl, $C_1$-$C_{12}$-Alkoxy- oder $OR^4$-Gruppe, $R^4$ ein Alkali-, Erdalkali- oder Ammonium-Ion oder eine $(R^5)_4N^+$-Gruppe, $R^5$ ein Wasserstoffatom oder eine $C_1$-$C_6$-Alkyl-Gruppe,

$R^2$ ein Wasserstoff- oder Deuteriumatom,

p null oder eine ganze Zahl von 1 bis 3 bedeutet,

aus einer am kern teilweise oder vollständig chlorierten und/oder bromierten Verbindung der Formel II

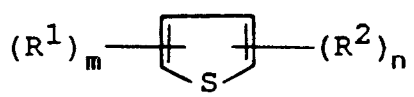

(II),

worin

$R^1$ und $R^2$ die vorgenannte Bedeutung haben und

m eine ganze Zahl von 1 bis 4 und

n null oder eine ganze Zahl von 1 bis 3 bedeuten, und m und n = 4 sind

durch elektrochemische Reaktion, das dadurch gekennzeichnet ist, daß man die elektrochemische Reaktion kontinuierlich oder diskontinuierlich in einer geteilten Elektrolysezelle in einer Elektrolysenflüssigkeit, bestehend aus Wasser oder einem oder mehreren organischen Lösemitteln und aus 1 bis 70 % einer Verbindung der Formel II, bezogen auf die Gesamtmenge des Katholyten, in Gegenwart mindestens einer Onium-Verbindung oder einer Verbindung, die im Elektrolyten in eine Onium-Verbindung umgewandelt wird, die mindestens ein Stickstoff- oder Phosphoratom enthält, und in Gegenwart von mindestens einem löslichen Salz eines Metalles mit einer Wasserstoffüberspannung größer als 0,25 V, bezogen auf eine Stromdichte von 100 mA/cm², an einer Kathode mit einer mittleren bis hohen Wasserstoffüberspannung bei einer Stromdichte von 10 bis 500 mA/cm² und bei einer Temperatur von 0 °C bis zur Siedetemperatur des Elektrolyten durchführt.

2. Verfahren nach Anspruch 1, das dadurch gekennzeichnet ist, daß als organische Lösemittel Methanol, Ethanol, die verschiedenen Propanole, Dichlormethan und halogenierten aromatische Verbindungen verwendet werden.

**3.** Verfahren nach Anspruch 1, das dadurch gekennzeichnet ist, daß Verbindungen der Formeln III bis VII

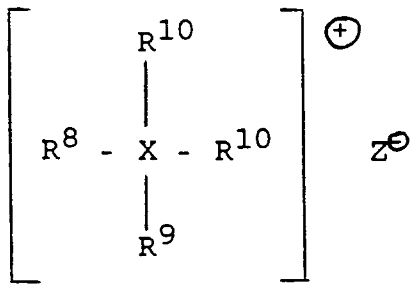

(III)

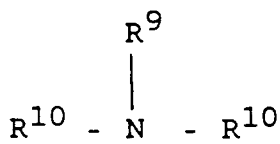

(IV)

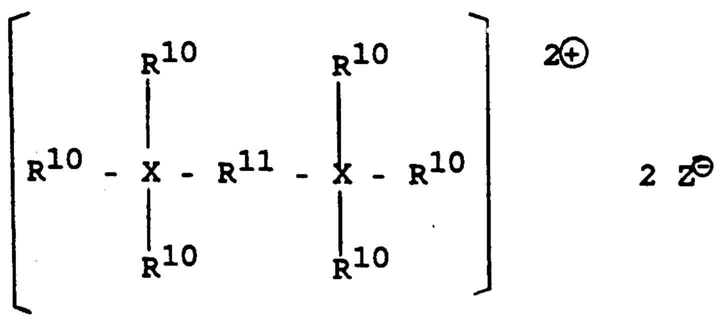

(V)

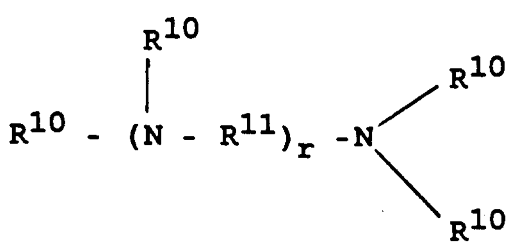

(VI)

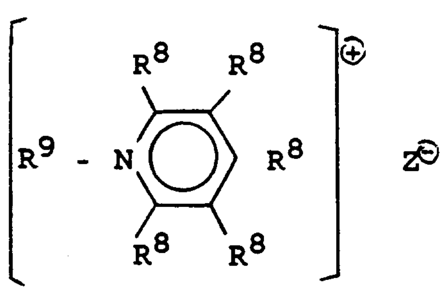

(VII),

worin

| | |
|---|---|
| X | Phosphor oder Stickstoff, |
| $R^8$ | Wasserstoff, Alkyl, Cycloalkyl, Aralkyl mit 1 bis 18 C-Atomen im Alkylrest und Aryl mit 6 bis 12 C-Atomen, |
| $R^9$ | gleich $R^8$ oder eine $(R^8\text{-}O)q$ $R^8$-Gruppe, q eine ganze Zahl von 1 bis 12, |
| $R^{10}$ | gleich $R^8$ oder $R^9$ oder bedeutet eine $-(CH_2Y)_r-CH_2$-Gruppe, r null oder eine ganze Zahl von 1 bis 6, Y -NH, Sauerstoff, Schwefel oder eine $-CH_2$-Gruppe |
| $R^{11}$ | $-(CH_2)_r-$, $-CH_2-[O-(CH_2)_r]_r-O-(CH_2)_2-$ und |
| Z | eine OH-Gruppe oder ein Anion einer anorganischen oder organischen Säure bedeuten, in |

Konzentrationen von $10^{-3}$ bis 10 %, bezogen auf die Gesamtmenge des Katholyten, verwendet werden.

**4.** Verfahren nach Anspruch 1, das dadurch gekennzeichnet ist, daß dem Katholyten Salze der Metalle Blei, Zink, Zinn, Cadmium oder Thallium in Konzentrationen von $10^{-3}$ bis 10 %, bezogen auf die Gesamtmenge des Katholyten, zugesetzt werden, wobei die Anionen $Cl^-$, $Br^-$, $SO_4^{2-}$, $NO_3^-$ $CH_3COO^-$ und $PO_4^{3-}$ darstellen.

**5.** Verfahren nach Anspruch 1, das dadurch gekennzeichnet ist, daß an Kathoden aus Blei, Zink, Zinn, Cadmium, Kupfer, Quecksilber oder Legierungen dieser Metalle oder Kohlenstoff elektrolysiert wird.

**6.** Verfahren nach Anspruch 1, das dadurch gekennzeichnet ist, daß in sauren Katholyten an einer Kathode aus Kohlenstoff elektrolysiert wird.

**7.** Verfahren nach Anspruch 1, das dadurch gekennzeichnet ist, daß als Kohlenstoffelektrode Elektrodengraphit, imprägnierter Graphit, poröser Graphit, Kohlefilze, glasartiger Kohlenstoff oder Kohlenstoff-Kunststoffverbundwerkstoffe eingesetzt werden.

**8.** Verfahren nach Anspruch 1, das dadurch gekennzeichnet ist, daß als Anolyt wäßrige oder alkoholische Lösungen von Halogenwasserstoffsäuren verwendet werden.

**9.** Verfahren nach Anspruch 1, das dadurch gekennzeichnet ist, daß in einer durch eine Kationen- oder Anionenaustauschermembran geteilten Zelle elektrolysiert wird.

## Claims

**1.** A process for the preparation of thiophene derivatives of the formula I

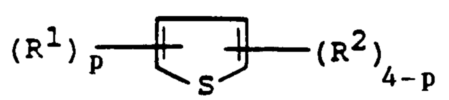

(I)

in which

$R^1$ is identical or different and is a $C_1$-$C_{12}$-alkyl, cycloalkyl, $C_6$-aryl, $C_1$-$C_{12}$-alkoxy, cycloalkoxy, $C_6$-aryloxy, $C_1$-$C_{12}$-alkylamino or halogenated $C_1$-$C_{12}$-alkyl group, a halogen atom or a CO-$R^3$ group, $R^3$ is a hydrogen atom, an OH, OD, $C_1$-$C_6$-alkyl, $C_1$-$C_{12}$-alkoxy or $OR^4$ group, $R^4$ is an alkali metal, alkaline earth metal or ammonium ion or a $(R^5)_4N^+$ group, $R^5$ is a hydrogen atom or a $C_1$-$C_6$-alkyl group,

$R^2$ is a hydrogen or deuterium atom, and

p is zero or an integer from 1 to 3,

from a compound of the formula II, which is partially or completely chlorinated and/or brominated in the nucleus,

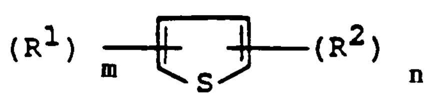

(II),

in which

$R^1$ and $R^2$ have the abovementioned meaning and

m is an integer from 1 to 4 and

n is zero or an integer from 1 to 3, and m and n = 4,

by electrochemical reaction, which comprises carrying out the electrochemical reaction continuously or batchwise in a divided electrolysis cell in an electrolysis liquid consisting of water or one or more organic solvents and of 1 to 70% of a compound of the formula II, relative to the total amount of the

catholyte, in the presence of at least one onium compound or a compound which is converted into an onium compound in the electrolyte, which contains at least one nitrogen or phosphorus atom, and in the presence of at least one soluble salt of a metal, having a hydrogen overvoltage of greater than 0.25 V, relative to a current density of 100 mA/$cm^2$, on a cathode having a medium to high hydrogen overvoltage, at a current density of 10 to 500 mA/$cm^2$ and at a temperature of 0 °C up to the boiling point of the electrolyte.

2. The process as claimed in claim 1, which comprises using methanol, ethanol, the various propanols, dichloromethane and halogenated aromatic compounds as organic solvents.

3. The process as claimed in claim 1, which comprises using compounds of the formulae III to VII

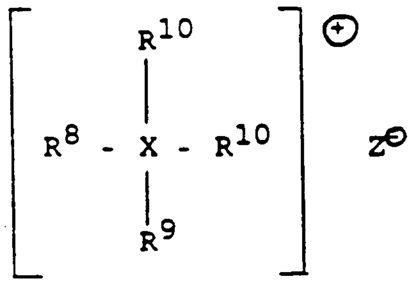

(III)

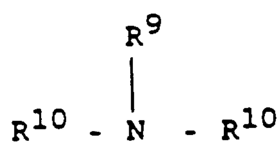

(IV)

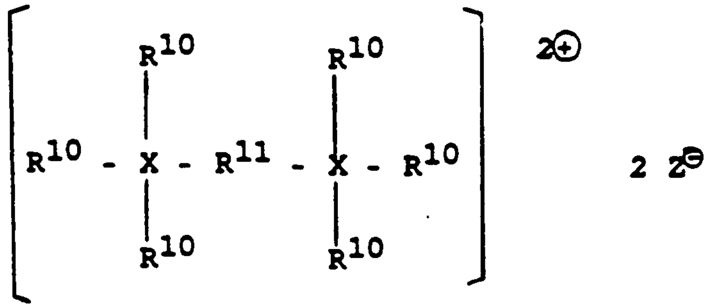

(V)

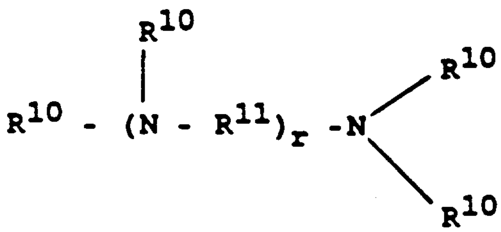

(VI)

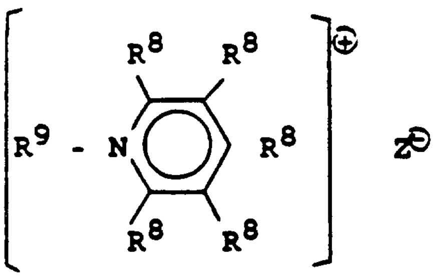

(VII)

in which

- X is phosphorus or nitrogen,
- $R^8$ is hydrogen, alkyl, cycloalkyl, aralkyl having 1 to 18 carbon atoms in the alkyl moiety and aryl having 6 to 12 carbon atoms,
- $R^9$ is identical to $R^8$ or is an $(R^8\text{-}O)_q$ $R^8$ group and q is an integer from 1 to 12,
- $R^{10}$ is identical to $R^8$ or $R^9$ or is a $-(CH_2Y)_r-CH_2-$group, r is zero or an integer from 1 to 6, and Y is -NH, oxygen, sulfur or a $-CH_2-$ group,
- $R^{11}$ is $-(CH_2)_r-$, $-CH_2-[O-(CH_2)_r]_r-O-(CH_2)_2-$ and
- Z is an OH group or an anion of an inorganic or

organic acid, in concentrations of $10^{-3}$ to 10%, relative to the total amount of the catholyte.

**4.** The process as claimed in claim 1, which comprises adding salts of the metals lead, zinc, tin, cadmium or thallium in concentrations of $10^{-3}$ to 10%, relative to the total amount of the catholyte, to the catholyte, the anions being $Cl^-$, $Br^-$, $SO_4^{2-}$, $NO_3^-$, $CH_3COO^-$ and $PO_4^{3-}$.

**5.** The process as claimed in claim 1, which comprises electrolyzing on cathodes of lead, zinc, tin, cadmium, copper, mercury or alloys of these metals or carbon.

**6.** The process as claimed in claim 1, which comprises electrolyzing in acidic catholytes on a cathode of carbon.

**7.** The process as claimed in claim 1, which comprises employing electrode graphite, impregnated graphite, porous graphite, carbon felts, vitreous carbon or carbon-synthetic composite materials as the carbon electrode.

**8.** The process as claimed in claim 1, which comprises using aqueous or alcoholic solutions of hydrohalic acids as the anolyte.

9. The process as claimed in claim 1, which comprises electrolyzing in a cell divided by a cation or anion exchange membrane.

**Revendications**

1. Procédé pour préparer des dérivés du thiophène de formule I

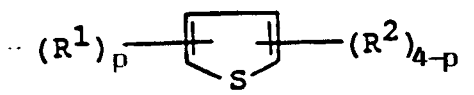

(I),

dans laquelle
$R^1$ représente, de façon identique ou différente, un alkyle en $C_1$-$C_{12}$, un cycloalkyle, un aryle en $C_6$, un alcoxy en $C_1$-$C_{12}$, un cycloalcoxy, un aryloxy en $C_6$, un alkylamino en $C_1$-$C_{12}$, un alkyle halogéné en $C_1$-$C_{12}$, un atome d'halogène ou un groupe CO-$R^3$, $R^3$ représente un atome d'hydrogène, un groupe OH, un groupe OD, un groupe alkyle en $C_1$-$C_6$, un groupe alcoxy en $C_1$-$C_{12}$, ou un groupe $OR^4$, $R^4$ représente un ion alcalin, un ion alcalino-terreux ou un ion ammonium ou un groupe $(R^5)_4N^+$, $R^5$ représente un atome d'hydrogène ou un groupe alkyle en $C_1$-$C_6$,
$R^2$ représente un atome d'hydrogène ou un atome de deutérium,
p représente zéro ou un nombre de 1 à 3,
à partir d'un composé de formule II, partiellement ou totalement chloré et/ou bromé sur le noyau,

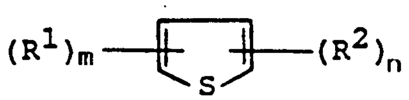

(II),

dans laquelle $R^1$ et $R^2$ ont la signification précédente et
m représente un nombre entier de 1 à 4 et
n est nul ou représente un nombre entier de 1 à 3 et
la somme de m et de n est égale à 4,
par réaction électrochimique, le procédé étant caractérisé en ce que l'on effectue la réaction électrochimique en continu ou en discontinu dans une cellule d'électrolyse divisée, dans un liquide d'électrolyse qui est constitué d'eau ou d'un ou plusieurs solvants organiques et de 1 à 70 % d'un composé de formule II, rapporté à la quantité totale du catholyte, en présence d'au moins un composé onium ou d'un composé qui est transformé en composé onium dans l'électrolyte, et qui comporte au moins un atome d'azote ou de phosphore, et en présence d'au moins un sel soluble d'un métal ayant une surtension d'hydrogène supérieure à 0,25 V, rapporté à une densité de courant de 100 mA/cm², sur une cathode ayant une surtension d'hydrogène de moyenne à élevée, à une densité de courant de 10 à 500 mA/cm² et à une température de 0° C jusqu'à la température d'ébullition de l'électrolyte.

2. Procédé selon la revendication 1, caractérisé en ce que l'on utilise comme solvant organique le méthanol, l'éthanol, les divers propanols, le dichlorométhane et les composés halogénés aromatiques.

3. Procédé selon la revendication 1, caractérisé en ce que l'on utilise des composés des formules III à VII

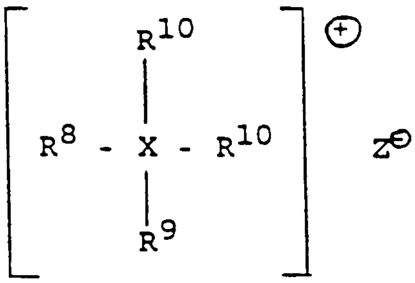

(III)

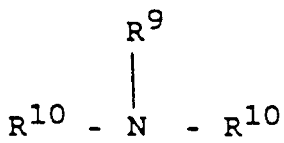

(IV)

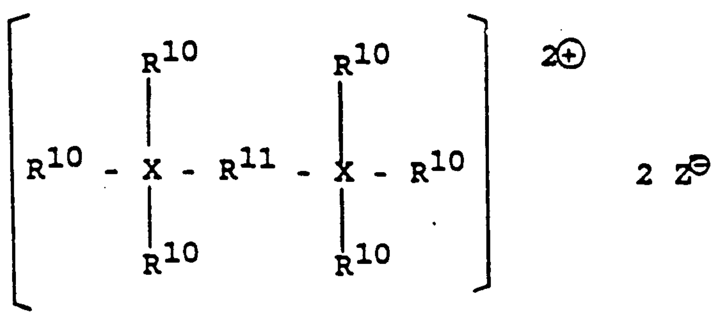

(V)

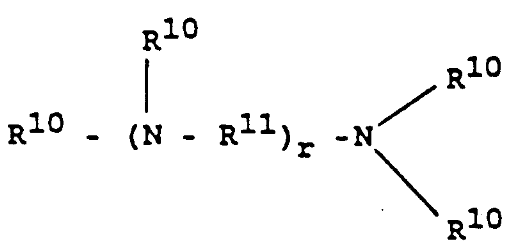

(VI)

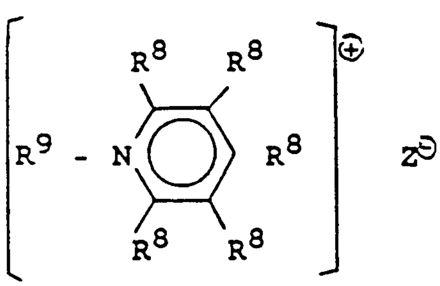

(VII),

dans lesquelles
X représente le phosphore ou l'azote,
$R^8$ représente l'hydroéne, un alkyle, un cycloalkyle, un aralkyle ayant de 1 à 18 atomes de C dans le radical alkyle et un aryle ayant de 6 à 12 atomes de C.
$R^9$ est identique à $R^8$ ou il représente un groupe $(R^8\text{-}O)_q\ R^8$ et q est un nombre entier de 1 à 12.
$R^{10}$ est identique à $R^8$ ou à $R^9$ ou il représente un groupe $-(CH_2Y)_r-CH_2-$, r est nul ou représente un nombre entier de 1 à 6, Y représente -NH, l'oxygène, le soufre ou un groupe-$CH_2$-.
$R^{11}$ est $-(CH_2)_r-$, $-CH_2-[-O-(CH_2)_r]_r-O-(CH_2)_2-$ et

Z est un groupe OH ou un anion d'un acide inorganique ou organique, dans des concentrations de $10^{-3}$ à 10%, calculées sur la quantité totale du catholyte.

4. Procédé selon la revendication 1, caractérisé en ce que l'on ajoute au catholyte des sels des métaux plomb, Zinc, étain, cadmium ou thallium, dans des concentrations de $10^{-3}$ à 10 %, calculées sur la quantité totale du catholyte, où les anions représentent $Cl^-$, $Br^-$, $SO_4^{2-}$, $NO_3^-$, $CH_3COO^-$ et $PO_4^{3-}$.

5. Procédé selon la revendication 1, caractérisé en ce que l'on effectue l'électrolyse sur des cathodes en plomb, en zinc, en étain, en cadmium, en cuivre, en mercure ou en alliages de ces métaux ou en carbone.

6. Procédé selon la revendication 1, caractérisé en ce que l'on effectue l'électrolyse dans des catholytes acides sur une cathode en carbone.

7. Procédé selon la revendication 1, caractérisé en ce que l'on utilise comme électrode de carbone du graphite pour électrode, du graphite imprégné, du graphite poreux, des feutres de carbone, du carbone vitreux ou des composites carbone-matière plastique.

8. Procédé selon la revendication 1, caractérisé en ce que l'on utilise comme anolyte des solutions aqueuses ou alcoolées d'hydracides halogéné.

9. Procédé selon la revendication 1, caractérisé en ce que l'on effectue l'électrolyse dans une cellule divisée par une membrane échangeuse de cations ou d'anions.